# EUROPEAN PATENT SPECIFICATION

(11) **EP 3 432 915 B1**
(45) Date of publication and mention of the grant of the patent: **22.04.2026**
(21) Application number: 17713637.1
(22) Date of filing: 24.03.2017
(51) Int. Cl.: A61K 38/48, A61P 25/00, A61P 21/02, A61P 43/00, G16H 20/17, G16H 50/20

(54) **COLLECTING PHYSICAL THERAPY INFORMATION TO ENHANCE TREATMENT EFFICACY OF BOTULINUM TOXIN**
ERFASSUNG VPN PHYSIOTHERAPIEINFORMATIONEN ZUR VERBESSERUNG DER BEHANDLUNGSWIRKSAMKEIT VON BOTULINUMTOXIN
COLLECTE D'INFORMATIONS DE THÉRAPIE PHYSIQUE POUR AMÉLIORER L'EFFICACITÉ DE TRAITEMENT DE LA TOXINE BOTULIQUE

(30) Priority: 25.03.2016 US 201662313418 P
(43) Date of publication of application: 30.01.2019
(73) Proprietor: Ipsen Biopharm Limited, Wrexham LL13 9UF (GB)
(72) Inventor: GRACIES, Jean-Michel, 75014 Paris (FR)
(74) Representative: MacLean, Martin Robert
(86) International application number: PCT/EP2017/057114
(87) International publication number: WO 2017/162870

(56) References cited:
- WO-A1-2013/022890
- WO-A1-2015/039244
- PRADINES MAUD ET AL: "Ultrasound Structural Changes in Triceps Surae After a 1-Year Daily Self-stretch Program: A Prospective Randomized Controlled Trial in Chronic Hemiparesis", NEUROREHABILITATION AND NEURAL REPAIR, vol. 33, no. 4, 1 April 2019 (2019-04-01), US, pages 245 - 259, XP055976470, ISSN: 1545-9683, DOI: 10.1177/1545968319829455
- GRACIES JEAN-MICHEL ET AL: "Guided Self-rehabilitation Contracts Combined With AbobotulinumtoxinA in Adults With Spastic Paresis", JOURNAL OF NEUROLOGIC PHYSICAL THERAPY, vol. 45, no. 3, 27 May 2021 (2021-05-27), pages 203 - 213, XP055976472, ISSN: 1557-0576, DOI: 10.1097/NPT.0000000000000359
- L. ANDREW KOMAN ET AL: "Upper Extremity Spasticity in Children With Cerebral Palsy: A Randomized, Double-Blind, Placebo-Controlled Study of the Short-Term Outcomes of Treatment With Botulinum A Toxin", THE JOURNAL OF HAND SURGERY, vol. 38, no. 3, 1 March 2013 (2013-03-01), AMSTERDAM, NL, pages 435 - 446.e1, XP055374536, ISSN: 0363-5023, DOI: 10.1016/j.jhsa.2012.12.019
- FRANCESCHINI: "Management of stroke patients submitted to botulinum toxin type A therapy: a Delphi survey of an Italian expert panel of specialist injectors.", 1 October 2014 (2014-10-01), XP055374649, Retrieved from the Internet <URL:http://www.ncbi.nlm.nih.gov/pubmed/24963604> [retrieved on 20170519]
- ULLA BERGFELDT ET AL: "Cortical activation changes and improved motor function in stroke patients after focal spasticity therapy? an interventional study applying repeated fMRI", BMC NEUROLOGY, BIOMED CENTRAL LTD., LONDON, GB, vol. 15, no. 1, 11 April 2015 (2015-04-11), pages 52, XP021222581, ISSN: 1471-2377, DOI: 10.1186/S12883-015-0306-4
- HOARE BRIAN J ET AL: "Modified constraint-induced movement therapy or bimanual occupational therapy following injection of Botulinum toxin-A to improve bimanual performance in young children with hemiplegic cerebral palsy: a randomised controlled trial methods paper", BMC NEUROLOGY, BIOMED CENTRAL LTD., LONDON, GB, vol. 10, no. 1, 5 July 2010 (2010-07-05), pages 58, XP021074841, ISSN: 1471-2377, DOI: 10.1186/1471-2377-10-58

## Description

### Field of the Invention

The present invention relates generally to improving active movement capacity in a subject, and more particularly, to using a prescribed physical therapy regimen in conjunction with a *Clostridial* neurotoxin, such as botulinum toxin, to reduce symptoms associated with impaired or abnormal active movement capacity.

### Background

Abnormal movement capacity includes muscle rigidity or stiffness, tightness, contracture, spasms (*i.e*., jerky involuntary movements), dystonia, hypertonia, and clonus (*i.e*., repetitive involuntary movement or reflex). Abnormal movement capacity is commonly associated with neurological disorders, including stroke, cerebral palsy, muscular dystrophy, spinal cord injury, brain injury, spastic disorders, such as blepharospasm, spasmodic torticollis (cervical dystonia), oromandibular dystonia and spasmodic dysphonia (laryngeal dystonia), and neurodegenerative diseases, such as multiple sclerosis and Parkinson's disease. It can also be associated with various other metabolic disorders, muscle diseases, upper or lower motor neuron lesions, and Guillain-Barré syndrome.

In addition to potentially causing pain, the symptoms associated with abnormal muscle tone can interfere with a person's voluntary movement and ability to carry out daily activities. Physical therapy with stretching and active exercise is a recognized treatment for abnormal muscle tone. Additional information regarding how physical therapy can be useful for treating movement capacity caused by neurologic movement disorders is described in Veerbeek, Janne Marieke et al., "What Is the Evidence for Physical Therapy Poststroke? A Systematic Review and Meta-Analysis," PLOS One, vol.9:2 (2014).

In addition to physical therapy, application of a toxin, such as botulinum toxin, to the affected muscles can be helpful. For example, when injected into a muscle, the botulinum toxin binds rapidly and strongly to presynaptic cholinergic nerve terminals and inhibits the exocytosis of acetylcholine from the nerve ending to the muscle fiber. This results in partial paralysis, and hence relaxation, of the muscle afflicted by spasm.

Botulinum toxin can be used to treat various types of neurologic conditions, including movement disorders associated with injury or disease of the central nervous system (such as trauma, stroke, multiple sclerosis, Parkinson's disease, and cerebral palsy), cervical dystonia (spasmodic torticollis), blepharospasm, hyperhidrosis, chronic daily headache, strabismus, esophageal achalasia, and focal dystonia. For additional information regarding how botulinum toxin can be useful in the treatment of spasticity, reference is made to Simpson D.M. et al, "Assessment: Botulinum neurotoxin for the treatment of spasticity (an evidence-based review): Report of the Therapeutics and Technology Assessment Subcommittee of the American Academy of Neurology," Neurology 6;70(19), 1691-1698 (2008). Koman et al (J Hand Surg Am. 2013 Mar;38(3):435-46.e1) describes upper extremity spasticity in children with cerebral palsy, and a randomized, double-blind, placebo-controlled study of the short-term outcomes of treatment with botulinum A toxin. Franceschini et al (Eur J Phys Rehabil Med. 2014 Oct;50(5):525-33) describes the management of stroke patients submitted to botulinum toxin type A therapy and a Delphi survey of an Italian expert panel of specialist injectors. WO 2015/039244 describes a system for obtaining and analyzing data for overall joint motion from a plurality of joints of a subject experiencing a movement disorder. Bergfeld et al (BMC Neurol. 2015 Apr 11:15:52. doi: 10.1186/s12883-015-0306-4) describes cortical activation changes and improved motor function in stroke patients after focal spasticity therapy and an interventional study applying repeated fMRI. Hoare et al (BMC Neurol. 2010 Jul 5:10:58. doi: 10.1186/1471-2377-10-58) describes modified constraint-induced movement therapy or bimanual occupational therapy following injection of Botulinum toxin-A to improve bimanual performance in young children with hemiplegic cerebral palsy and a randomised controlled trial methods paper. WO 2013/022890 describes a system, apparatus and method for the non-invasive motion tracking to augment patient administered physical therapy.

### Summary of the Invention

The invention is defined by the claims. The invention provides a botulinum neurotoxin for use according to claim 1.

The botulinum toxin treatment(s) may be administered or received into the affected muscle, for example by injection. The treatment(s) may also be administered or received into a neuromuscular junction of the affected muscle, for example by injection.

In some embodiments, the physical therapy information is received by a software program that operates on a first computer device and physical therapy information is stored in a database on the first computer device or a second computer device that is in communication with first computer device.

In another embodiment of the invention, a computer system as defined by claim 7 is provided.

In another embodiment, a non-transitory computer-readable storage medium as defined by claim 8 is provided.

### Description of the Drawings

FIG. 1 shows a subject 10 being administered a dose of botulinum toxin by injection 12.
FIG. 2 shows the subject 10 entering physical therapy information into a tablet computer 14, which is in communication with a host server computer 22 via a network link 20.
FIG. 3 shows a medical practitioner 30 operating a computer 32 that is in communication via a communication link 24 that is in communication with a host computer system 22 that stores the information entered by the subject.

### Detailed Description of the Invention

The present invention is useful in the clinical management of a subject having impaired active movement capacity. Impairments in active movement capacity may be the result, for example, of abnormal muscle overactivity in the subject. The subject has a medical condition associated with impaired active movement capacity, selected from stroke, cerebral palsy, muscular dystrophy, spinal cord injury, brain injury, spastic disorders, such as blepharospasm, spasmodic torticollis (cervical dystonia), oromandibular dystonia and spasmodic dysphonia (laryngeal dystonia), and neurodegenerative diseases, such as multiple sclerosis and Parkinson's disease.

The affected muscle can be anywhere in the subject's body, including in the upper limbs (adult or pediatric) such as the shoulders, arms, or hands; in the lower limbs (adult or pediatric), such as in the leg or foot; or in the bladder (*e.g*., as affected in neurogenic detrusor overactivity (NDO). The term "affected muscle" as used herein refers to any muscle affected by impaired active movement capacity. With the proper treatment, the subject may experience various improvements in active movement capacity, such as increased mobility, increased flexibility, increased strength, increased passive or active range of motion in the affected limbs, reduced pain, and ability to independently perform activities of daily living.

In the present invention, a subject is administered botulinum toxin in conjunction with specifically prescribed physical therapy techniques. The administration of botulinum toxin is typically performed by a medical professional. As used herein, the term "medical professional" includes a clinician, physician, nurse, medical technician, or the like. In some embodiments, the subject may self-administer the botulinum toxin. The botulinum toxin can be administered in any suitable manner, such as by transdermal administration or injection into the affected muscle(s) or the neuromuscular junction of the affected muscle(s).

In the present invention, the botulinum toxin-producing strain is preferably *Clostridium* botulinum, but is not limited thereto, and it will be apparent to those skilled in the art that any strain capable of producing a botulinum toxin may be used in the present invention. As used herein, the term "botulinum toxin" is meant to include not only a neurotoxin produced by the *Clostridium* botulinum strain, but also any modified, recombinant, hybrid, fusion, and chimeric botulinum toxins. A modified or recombinant botulinum toxin may have a light chain and/or heavy chain produced by non-*Clostridium* species in a recombinant manner. In addition, the term "botulinum toxin" as used herein is meant to include any and all known botulinum toxin serotypes, including serotypes A, A1, A2, A3, A4, B, C, C1, D, E, F and G, as well as botulinum toxin complexes (e.g., 300, 600 and 900 kDa complexes), and a pure botulinum toxin (e.g., a 150 kDa neurotoxic molecule), which are all useful in the practice of the present invention. For additional information regarding the properties of the various botulinum toxins, reference is made to Simpson D.M. et al, "Assessment: Botulinum neurotoxin for the treatment of spasticity (an evidence-based review): Report of the Therapeutics and Technology Assessment Subcommittee of the American Academy of Neurology," Neurology 6;70(19), 1691-1698 (2008).

In one embodiment of the invention, the botulinum toxin administered to the subject is type A toxin. Botulinum toxin type Al complex is marketed, e.g., under the trade names DYSPORT^{®}, XEOMIN^{®}, CORETOXO, RELOXIN^{®}, and BOTOX^{®}. In another embodiment of the invention, the botulinum toxin used is type B toxin, e.g., as marketed under the trade names MYOBLOC^{®} and NEUROBLOC^{®}. In other embodiments of the invention, the botulinum toxin used is any of the other known toxin types, including A1, A2, A3, A4, C, C1, D, E, F or G.

Botulinum toxin is obtained commercially by establishing and growing cultures of *C*. *botulinum* in a fermenter, and harvesting and purifying the fermented mixture in accordance with known techniques. The "A" form of botulinum toxin is currently available commercially from several sources, for example, from Ipsen Biopharmaceuticals Limited under the tradename DYSPORT^{®}, from Merz Pharma under the tradename XEOMIN^{®}, from Medytox Inc. under the tradename CORETOX^{®}, and from Allergan Inc. under the tradename BOTOX^{®}.

The biological activity of botulinum toxin relates, e.g., to inhibition of neurotransmission over the synapse at the neuromuscular junction, leading to muscle paralysis or inhibition of exocytosis, in particular exocytosis of acetylcholine or of another neurotransmitter. The biological activity of botulinum neurotoxin is linked to its proteolytic activity. One way to determine the biological activity of any botulinum toxin is, therefore, to measure the proteolytic activity on the relevant substrate mentioned above. Assays that can be used to determine this activity are known in the art; one such assay is described in WO 95/33850.

The botulinum toxin may be administered by any means known in the art, including injection directly into an affected muscle (i.e., intramuscular injection), injection into the neuromuscular junction of the affected muscle, sub-cutaneous injection, or transdermal administration.

Targeting of neuromuscular junctions can increase the effectiveness of the botulinum toxin treatment and/or allow for use of lower concentrations dosages of botulinum toxin, as described in Gracies JM, Lugassy M, Weisz DJ, Vecchio M, Flanagen S, Simpson DM, "Botulinum toxin dilution and endplate targeting in spasticity: a double-blind controlled study," Arch Phys Med Rehabil 2009, 90: 9-16. In some embodiments, the botulinum toxin is injected into a neuromuscular junction.

Transdermal administration allows the toxin to be delivered to a target site associated with impaired active movement capacity to provide a therapeutic effect, such as a relaxation of the muscle or a decrease in muscle spasticity, without the difficulty and discomfort associated with needle injection of the botulinum toxin. If desired, adhesive patches containing amounts of a botulinum toxin sufficient to improve active movement capacity can be self-administered by the subject based on a medical practitioner's instructions. Use of an adhesive patch for transdermal delivery of a therapeutic drug is described, for example, in Tonnesen, P. et al., "A double blind trial of a 16-hour transdermal nicotine patch in smoking cessation," New Eng J Medicine, 325(5); 311-315: August 1991.

The botulinum toxin can be presented as a sterile pyrogen-free aqueous solution or dispersion, or as a sterile powder for reconstitution into a sterile solution or dispersion. If desired, tonicity adjusting agents, such as sodium chloride, glycerol and/or various sugars can be added. Stabilizers may be included if desired. The formulation may be preserved by means of any suitable pharmaceutically acceptable preservative, such as a paraben.

In some embodiments, the botulinum toxin is formulated in unit dosage form, for example, as a sterile solution in a vial, or as a vial or sachet containing a lyophilized powder for reconstituting in a suitable carrier, such as saline, for injection. In one aspect, the botulinum toxin is formulated in a solution containing saline and pasteurized human serum albumin, which stabilizes the toxin. The solution may be sterile filtered, filled into individual vials, and then vacuum dried to give a sterile lyophilized powder. In use, the powder can be reconstituted by the addition of sterile unpreserved normal saline (sodium chloride 0.9% for injection). In another aspect, the commercially available sterile botulinum toxin powder can be incorporated into the polymeric matrix of a suitable carrier using known methodologies, and formed into an adhesive patch for use in conjunction with a skin permeation enhancer such as dimethyl sulfoxide (DMSO) or Azone (1-dodecylazacycloheptan-2-one).

Typically, the amount of the botulinum toxin administered to the subject is sufficient to improve the impairment in active movement capacity in the one or more affected muscles. The amount of the botulinum toxin will depend upon a variety of factors, including the severity of the condition, the number of muscle groups requiring treatment, the age, size, and/or gender of the subject, and the type and potency of the particular toxin. The potency of the toxin may be expressed as a multiple of the LD₅₀ value. One LD₅₀ unit is the equivalent amount of toxin which causes the death of 50% (one-half) of a group of test animals, such as laboratory mice. Alternative methods of determining the potency of the toxin may also be employed, including, for example, any method included in the *European Pharmacopoeia* monograph 01/2005:2113.

The dose administered to the subject is in an amount effective to improve active movement capacity. For example, the amount may be between 0.01 and 1000 units of botulinum toxin, whatever the type of botulinum toxin or whatever its provenance. Smaller or larger doses may be administered in appropriate circumstances. In some embodiments, the dosage amount of the botulinum toxin is from about 1 to about 500 units per muscle injection. For example, the dosage amount could be about 1 unit, 50 units, 100 units, about 150 units, about 200 units, about 250 units, about 300 units, about 350 units, about 400 units, about 450 units, or about 500 units. In one embodiment, a subject with impaired active movement capacity (e.g., a patient having spastic paresis) is administered up to 1000 units of abobotulinum toxin A (DYSPORT^{®}). Additional information regarding appropriate dosage amounts of botulinum toxin are described in the publications by Jabeen, Afshan et al., "Guidelines for the use of botulinum toxin in movement disorders and spasticity," Ann Indian Acad Neurol., vol. 14 (Suppl 1), pp. S31-S34: July 2011 and by Ozcakir, Suheda et al., "Botulinum Toxin in Poststroke Spasticity," Clin Med Res., vol. 5(2), pp. 132-138: June 2007.

Depending on the potency of the botulinum toxin, and its duration of action, the doses may need to be administered intermittently. Ultimately, however, both the quantity of toxin administered, and the frequency of its administration will be at the discretion of the medical practitioner(s) responsible for the treatment, and will be commensurate with questions of safety and the effects produced by the toxin.

Recent studies have demonstrated that subjects experiencing post-stroke spasticity can be benefited by receiving early botulinum toxin treatment. For example, *see* Verplancke et al., "A randomized controlled trial of botulinum toxin on lower limb spasticity following acute acquired severe brain injury," Clin Rehabil 2005; 19:117-125; Cousins et al., "Does low-dose botulinum toxin help the recovery of arm function when given early after stroke? A phase II randomized controlled pilot study to estimate effect size," Clin Rehabil 2010;24:501-513; Hesse et al., "An early botulinum toxin A treatment in subacute stroke patients may prevent a disabling finger flexor stiffness six months later: a randomized controlled trial," Clin Rehabil 2012;26:237-245; Rosales et al., "Botulinum toxin injection for hypertonicity of the upper extremity within 12 weeks after stroke: A randomized controlled trial," Neurorehabil Neural Repair 2012;26:812-821; and Fietzek et al., "Early botulinum toxin treatment for spastic pes equinovarus-a randomized double-blind placebo-controlled study," Eur J Neurol 2014;21:1089-1095.

Accordingly, in some embodiments of the method, a subject experiencing symptoms of impaired active movement capacity (e.g., spasticity) arising from a stroke receives early botulinum toxin treatment, e.g., within one year of the stroke. For example, a subject who has suffered a stroke may be administered his or her first botulinum toxin treatment within 9 months, 6 months, 3 months, 2 months, 1 month, 3 weeks, 2 weeks, 1 week, or even a few days after suffering the stroke.

Successful treatment of impaired active movement capacity with botulinum toxin can be determined using routine methods known to persons of ordinary skill in the art. For example, successful treatment can be associated with improved active movement capabilities, reduced muscle tone, reduced pain, reduced spasticity, reduced deformity, and the like. The determination can be made by the subject, one or more medical professionals, or a combination of the two.

In the present invention, in conjunction with the botulinum toxin treatment, the subject engages in physical therapy as defined in the claims.

Additional information regarding the types of physical therapy activities that can be useful for treating neurologic movement disorders are described in Veerbeek, Janne Marieke et al., "What Is the Evidence for Physical Therapy Poststroke? A Systematic Review and Meta-Analysis," PLOS One, vol. 9:2, e87987: Feb. 2014.

In some embodiments, a treatment regimen will be determined for the subject. As used herein, the term "treatment regimen" means the one or more types of treatments the subject is undergoing to improve active movement capacity, including botulinum toxin treatments, physical therapy, or a combination of the two.

In one embodiment, the treatment regimen will be determined based, at least on part, on the personal work and the regular reports by the subject (e.g., a patient). In one aspect, the subject is treated according to a Guided Self-rehabilitation Contract (GSC). This strategy aims to generate and maintain patient motivation, so as to enable long-term and intense use of physical therapy techniques, such as stretching and training. This may produce substantial functional improvements in chronic stages. In such contracts, the therapist acts as a coach, providing for example: technical guidance by selecting and teaching required exercises to the patient in infrequent, thorough visits (for example, every month) for a duration of at least one year; and psychological support to encourage compliance by the patient. In some embodiments, the subject visits the medical practitioner at least two or more times over the course of one year; in some cases, three or more times over the course of one year; in some cases, four or more times over the course of one year; in some cases, five or more times over the course of one year; and in some cases, six ormore times over the course of one year.

The patient, in turn, agrees to perform the prescribed daily stretch postures and maximal amplitude alternating movements over the long term and documents this work in a written diary. To facilitate such contracts, a manual and an application for cell-phones and tablets can be used. Additional information regarding the strategy of Guided Self-rehabilitation Contracts is described in Gracies et al., "Contrat d'Autorééducation Guidée dans la parésie spastique," Association Neuroloco, Paris, ISBN 978-2-35327-169-6 (2013) and Gracies et al., "The Concept of Guided Self-Rehabilitation Contracts in the Treatment of Deforming Spastic Paresis, Physikalische Medizin Rehabilitationsmedizin Kurortmedizin 25(03) (2015).

In some embodiments, the subject is under regular guidance by a medical practitioner and/or therapist. The medical practitioner and/or therapist may select and teach the physical activity to the subject. The medical practitioner and/or therapist may also offer encouragement to the subject, which in turn leads to improved adherence to the physical therapy and contributes to the subject's improved active movement capacity.

In some embodiments of the invention, the clinical management of the subject's impaired active movement capacity is enhanced by virtue of the fact that information about the physical therapy is being recorded as claimed.

In some aspects of the invention, the subject's motivation is improved as a result of recording the physical therapy information. As used herein, the term "motivation" refers to the subject's desire or willingness to continue to engage in physical therapy. The act of recording the physical therapy information may, for example, result in the subject being more likely to continue to engage in physical therapy, or to engage in more physical therapy, or for a longer period of time. In some embodiments, the act of recording the physical therapy information results in the subject actually engaging in more physical therapy than he or she would have engaged in had he or she not recorded the physical therapy information. In some embodiments, the subject's improved motivation resulting from recording the physical therapy information is responsible, at least in part, for the improvement in the subject's active movement capacity.

In some aspects, the improvement in the subject's active movement capacity is substantial. Techniques for measuring improvement in active movement capacity are well known in the art, and include, for example, the Modified Ashworth Scale (MAS), Tardieu Scale (TS), and Triple Spasticity Scale (TSS). Additional information regarding how to measure active movement capacity is described in Li et al, "Reliability of a new scale for measurement of spasticity in stroke patients," J. Rehabil. Med. 46(8), 746-53 (2014).

In some embodiments, the physical therapy is tailored to the particular subject. The subject is instructed to undergo physical therapy and/or is instructed to record information associated with the physical therapy at a regular interval, such as three times a day, twice a day, or once a day (daily). As a result, the subject may engage in physical therapy and/or record the information associated with the physical therapy at a regular interval, such as three times a day, twice a day, or once a day (daily).

A software program is used in conjunction with the botulinum toxin treatment and/or physical therapy, such as the GSC strategy. In these embodiments, a medical practitioner and/or therapist instructs the subject to use a software program that receives information associated with the physical therapy. The software program is running on any suitable computer device. Various sorts of information may be received by the software program, such as the types of physical therapy activities performed, duration of the physical therapy activities, frequency of the physical therapy activities, symptoms relating to the impaired active movement capacity (e.g., amount of pain or stiffness, frequency of muscle spasms, duration of muscle spasms, range of motion, etc.), the effect (if any) of the physical therapy activities, and any other comments about the physical therapy activities. The software program receives the information that is associated with the subject's physical therapy and stores that information into a database. The database can reside on the same computer device used by the subject or on a different computer device that is able to communicate with the computer device used by the subject.

Information associated with the physical therapy is used to determine or vary the mode, amount, or frequency or select the muscle for a second dose of the botulinum neurotoxin treatment.

As used herein, the term "optimal treatment regimen" means the treatment regimen determined by the medical practitioner to be optimal for a particular subject based on a variety of factors, including the age, size, and/or gender of the subject, the muscle group(s) requiring treatment, the potency of the toxin, the physical therapy activities performed by the subject, the duration and frequency of those physical therapy activities, the subject's symptoms relating to the impaired active movement capacity, and the information associated with the physical therapy activities. The optimal treatment regimen can comprise administration of botulinum toxin to the affected muscle, prescription of physical therapy activities (e.g., within a GSC strategy), or a combination of the two. In one aspect, the optimal treatment regimen comprises a prescription for physical therapy followed by administration of botulinum toxin in an amount sufficient to improve active movement capacity in the one or more affected muscles.

As used herein, the term "computer device" refers to any electronic device for storing and processing data, typically in binary form, according to instructions given to it in a software program, and includes, for example, a desktop, laptop, or tablet personal computers; "netbooks"; mobile communication devices, such as smartphones; personal digital assistants; portable audio or video file players; portable game players; portable electronic readers; or equivalent devices. The computer device can be in communication with another computer device by any suitable type of network (such as internet), and can use any suitable protocol, medium (e.g. fiber optic, coaxial cable, wireless broadband, etc.), network interface, or bandwidth.

The computer device used by the subject can be the same or different from the computer device used by the medical practitioner to access the physical therapy information. In some embodiments, the computer device used by the subject is the same computer device that the medical practitioner uses to access the physical therapy information. For example, the subject may enter the physical therapy information into his/her smartphone and bring that smartphone to the clinic visit to have the information viewed by the medical practitioner directly from the smartphone. In other embodiments, the computer device that the medical practitioner uses to access the physical therapy information is different from the computer device used by the subject to enter the physical therapy information. For example, the subject may enter the physical therapy information into a website portal that is specially designed to collect this type of information and the medical practitioner uses his/her own desktop computer to access the website portal and view the information that the subject has entered. In another example, the subject may be wearing a portable electronic device (*e.g*., a smart-watch or body-mounted exercise tracker), which records the subject's physical therapy activity and transmits the information to the subject's computer device.

The computer device on which the database resides may be the same or different from the computer device used by the subject to receive the physical therapy information and/or the computer device used by the medical practitioner to access that information. In some embodiments, the computer device on which the database resides is different from both the computer device used by the subject to receive the physical therapy information and the computer device used by the medical practitioner to access that information. For example, the subject may enter the information into a website portal via their laptop computer. The information is stored on a web server computer, which is then accessed by the medical practitioner on his/her own computer to view the physical therapy information.

As used herein, the term "software product" refers to a non-transitory computer-readable storage medium storing instructions that when executed by a computer system, causes the computer system to perform the recited steps. The software product may reside on any suitable computer-readable storage medium, such as CD-ROM, DVD, memory, hard disk, flash drive, RAM, ROM, cache, and the like. The software platform for implementing the present invention can vary depending on design considerations such as user preference, cost, implementation, ease of use, machine capabilities, network limitations, etc.

In some embodiments, a computer system as claimed is provided.

The hardware platforms used by the subject, the medical practitioner, and/or any other third parties may be different, but operate together as a system. For example, the subject being treated could use his/her own computer device to enter the physical therapy information, that information could be stored on a different computer device located remotely (e.g., a third party web server), and the medical practitioner could use his/her own computer device to access the information. In this scenario, these three computer devices can be considered to operate together as a system. The physical and/or functional components of the computer system may be distributed, centralized, or arranged in any suitable manner. Communications between the different physical and/or functional component may be performed in any suitable way. Moreover, the present invention encompasses all the various ways in which the operating work may be divided among different physical and/or functional components.

In some embodiments, the physical therapy information is received by the computer system from a first computer device, the database resides on a second computer device, and the physical therapy information is transmitted by the computer system to a third computer device. The computer devices may be separate. The first computer device may be in communication with the second computer device and the second computer device may be in communication with the third computer device. In one aspect, the first computer device is programmed to receive the physical therapy information from the subject, the database resides on a second computer device, and the third computer device receives the transmitted physical therapy information.

In other embodiments, a non-transitory computer-readable storage medium as claimed storing instructions is provided.

To assist in understanding the present invention, a particular embodiment is described in detail with references to the figures.

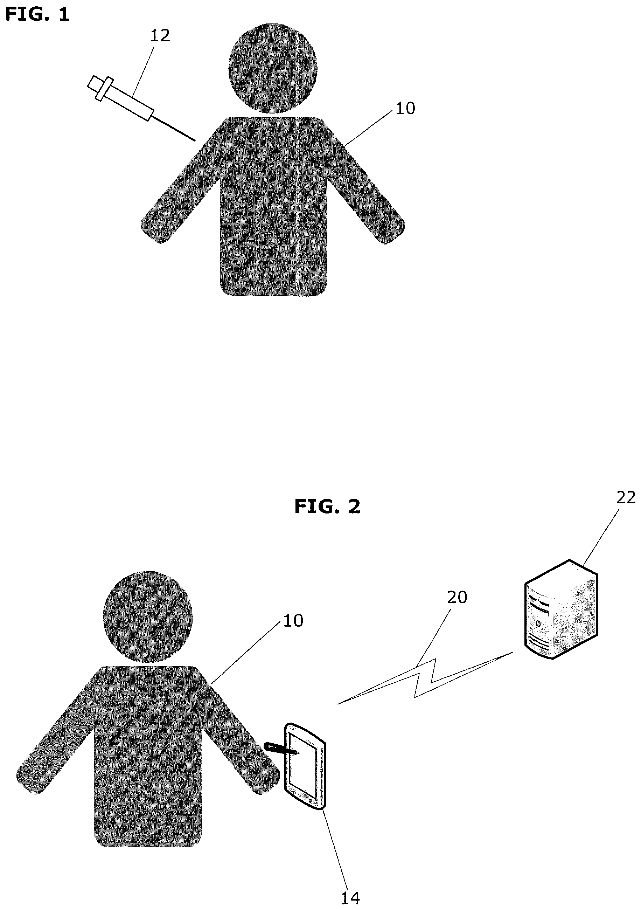

FIG. 1 shows a subject 10 being administered a dose of botulinum toxin by injection (e.g., by a syringe needle 12) into an overactive muscle. This injection could be performed in any suitable clinical setting, such as at a hospital or in an outpatient clinic. The medical practitioner 30 determines which muscle(s) need to be injected, the dosage amount, etc. If needed, multiple injections can be performed.

In conjunction with the botulinum toxin treatment (e.g., after administering the injection), the medical practitioner 30 instructs the subject 10 to undergo physical therapy activities and to use a computer software program to enter information associated with those physical therapy activities. The subject 10 undergoes the physical therapy as instructed by the medical practitioner 30. The physical therapy can be performed in any suitable manner, and performed directly by the subject 10 himself/herself (c.g., the subject 10 self-performs exercises, stretching, etc. at home), or a combination thereof.

FIG. 2 shows the subject 10 entering information associated with the physical therapy into a tablet computer 14. Installed on the tablet computer 14 is an application program that provides a user interface for the subject 10 and that receives the information entered by the subject 10. The tablet computer 14 is in communication with a host server computer 22 (e.g., operated by the supplier of the software program, or the supplier of the botulinum toxin, or another third party) via a network link 20 (e.g., connection through WiFi and then through a home internet connection). The information entered by the subject 10 is transmitted to the host server computer 22 and stored in a database residing therein.

In one embodiment, the subject's 10 interaction with the software program alone is sufficient to enhance the efficacy of the treatment. For example, in the GSC strategy, the act of entering information associated with the physical therapy activities performed by the subject 10 could give the subject 10 a feeling of accomplishment and encourage him/her to continue with the physical therapy activities, thereby enhancing the efficacy of the treatment.

The physical therapy information is used as defined in the claims.

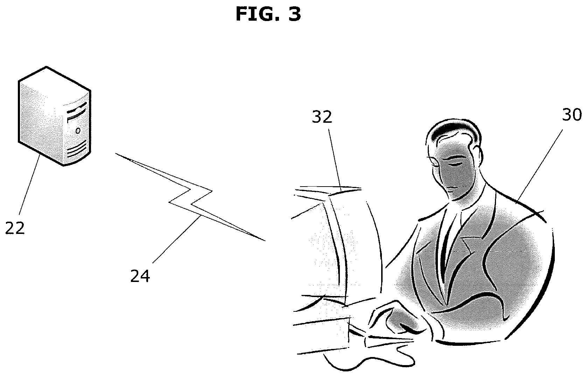

FIG. 3 shows a medical practitioner 30 working on a personal computer 32 that is in communication via a communication link 24 (e.g., by an office internet connection) with the host server computer 22. Using the personal computer 32, the medical practitioner 30 is able to access the subject's 10 physical therapy information stored on host server computer 22 and view the information. In an alternate embodiment, the subject 10 could bring the tablet computer 14 to the medical practitioner 30, who then views the information directly on the tablet computer 14.

Based on review of this information, the medical practitioner 30 may perform any suitable action to improve the clinical management of the subject's 10 impaired active movement capacity. For example, the medical practitioner could inform the subject 10 that he/she is pleased to see the progress made by the subject 10 and give encouragement to continue with the physical therapy. In an alternate embodiment, the medical practitioner could use this information to make decision(s) about further treatment of the subject with botulinum toxin. For example, the subject 10 may be receiving botulinum toxin injections intermittently at regular intervals (e.g., every three months) and, after reviewing the subject's 10 physical therapy information, the medical practitioner 30 may decide to make adjustments to the botulinum toxin treatment, such as adjusting the mode of administration, the frequency, or the amount of each dose, or selecting which muscle(s) to administer the botulinum toxin.

The medical practitioner 30 can access and review the subject's 10 physical therapy information during the subject's 10 clinic visit or in preparation for the subject's 10 next clinic visit. Having reviewed the physical therapy information and making a decision to adjust the botulinum toxin treatment, the medical practitioner 30 (or another medical professional) may administer the botulinum toxin to the subject 10 according to the adjustment in mode, dosage amount, frequency, muscle selection, etc.

## Claims

1. A botulinum neurotoxin for use in combination with physical therapy in a method for improving active movement capacity in a subject that has a medical condition associated with impaired active movement, the method comprising:
administering to a subject in need thereof a first botulinum neurotoxin treatment, wherein the botulinum neurotoxin is administered to a muscle affected by impaired active movement capacity; and
(a) instructing the subject to undergo physical therapy that comprises daily self-stretching the affected muscle; and (b) instructing the subject to record information associated with the physical therapy for review by a medical practitioner;
wherein the subject undergoes the physical therapy that comprises daily self-stretching the affected muscle and the subject records the information associated with the physical therapy into a software program daily;
wherein recording the information associated with the physical therapy enhances the improved active movement capacity associated with the botulinum toxin treatment; and
administering a second dose of the botulinum toxin treatment to the subject wherein said information associated with the physical therapy is used to determine or vary the mode, amount, or frequency or select the muscle for the second dose of the botulinum neurotoxin treatment;
wherein said medical condition is selected from stroke, cerebral palsy, muscular dystrophy, spinal cord injury, brain injury, a spastic disorder, and a neurodegenerative disease.

2. The botulinum neurotoxin for use according to claim 1, wherein said information associated with the physical therapy comprises the type of physical therapy activity performed; duration of the physical therapy activity; frequency of the physical therapy activity; symptoms relating to the impaired active movement capacity including amount of pain, amount of stiffness, frequency of muscle spasms, duration of muscle spasms, and/or range of motion; and/or the effect of the physical therapy activity.

3. The botulinum neurotoxin for use of any one of the preceding claims, wherein impairments in active movement capacity in the subject are caused by abnormal muscle overactivity.

4. The botulinum neurotoxin for use of any one of the preceding claims, wherein
a. the administration of the botulinum neurotoxin treatment is by injection into the affected muscle;
b. the step of administering the botulinum neurotoxin treatments comprises injecting botulinum neurotoxin into a neuromuscular junction;
c. the physical therapy information comprises the type of physical activity performed, duration of the physical activity, frequency of the physical activity, and/or symptoms relating to the impaired active movement capacity;
d. the physical therapy information is received on a first computer device and is accessed on a second computer device;
e. the subject has suffered from a stroke, and wherein the administration of the botulinum neurotoxin treatment occurs within three months of the stroke; and/or
f. recording the physical therapy information comprises entering the physical therapy information into a software program that is configured to receive the information;
optionally wherein the software program is operating on a computer device; and/or optionally wherein the physical therapy information is stored in a database.

5. The botulinum neurotoxin for use of any one of the preceding claims, wherein the subject is under regular guidance by a medical practitioner.

6. The botulinum neurotoxin for use of any one of the preceding claims, wherein:
the receiving of the first botulinum neurotoxin treatment occurs within three months of a stroke.

7. A computer system programmed to perform steps of a computer-implemented method, the method comprising:
receiving information associated with a physical therapy, from a subject undergoing said physical therapy and a botulinum neurotoxin treatment for impaired active movement capacity ;
storing the physical therapy information into a database; and
transmitting the physical therapy information to a medical practitioner who has administered the botulinum neurotoxin treatment to the subject;
wherein the subject has a medical condition associated with said impaired active movement,
wherein said medical condition is selected from stroke, cerebral palsy, muscular dystrophy, spinal cord injury, brain injury, a spastic disorder, and a neurodegenerative disease.

8. A non-transitory computer-readable storage medium storing instructions that, when executed by a computer system, causes the computer system to perform steps of a computer-implemented method, the method comprising:
receiving information associated with a physical therapy, from a subject undergoing said physical therapy and a botulinum neurotoxin treatment for impaired active movement capacity;
storing the physical therapy information into a database; and
transmitting the physical therapy information to a medical practitioner who has administered the botulinum neurotoxin treatment to the subject;
wherein the subject has a medical condition associated with said impaired active movement,
wherein said medical condition is selected from stroke, cerebral palsy, muscular dystrophy, spinal cord injury, brain injury, a spastic disorder, and a neurodegenerative disease.

9. The computer system of claim 7, wherein the physical therapy information is received by the computer system from a first computer device, the database resides on a second computer device that is part of the computer system, and the physical therapy information is transmitted by the computer system to a third computer device; optionally wherein the first, second, and third computer devices are each separate computer devices.

10. The computer system of claim 7 or 9, wherein the computer system comprises:
a first computer device that is programmed to receive the physical therapy information from the subject;
a second computer device on which the database resides; and
a third computer device which receives the transmitted physical therapy information; optionally
wherein the first, second, and third computer devices are each separate computer devices;
the first computer device is in communication with the second computer device; and
the third computer device is in communication with the second computer device.

## Patentansprüche

1. Botulinum-Neurotoxin zur Anwendung in Kombination mit Physiotherapie in einem Verfahren zum Verbessern der aktiven Bewegungsfähigkeit eines Patienten, der eine Erkrankung, die einer beeinträchtigten aktiven Bewegung zugeordnet ist, hat, wobei das Verfahren Folgendes umfasst:
Verabreichen einer ersten Botulinum-Neurotoxin-Behandlung an einen Patienten, der dessen bedarf, wobei das Botulinum-Neurotoxin in einen Muskel verabreicht wird, der von einer beeinträchtigten aktiven Bewegungsfähigkeit betroffen ist; und
(a) Anweisen des Patienten, sich einer Physiotherapie zu unterziehen, die ein tägliches Selbst-Dehnen des betroffenen Muskels umfasst; und (b) Anweisen des Patienten, Informationen, die der Physiotherapie zugeordnet sind, zur Überprüfung durch einen Arzt, aufzuzeichnen;
wobei sich der Patient der Physiotherapie unterzieht, die das tägliche Selbst-Dehnen des betroffenen Muskels umfasst, und der Patient die Informationen, die der Physiotherapie zugeordnet sind, täglich mithilfe eines Softwareprogramms aufzeichnet;
wobei das Aufzeichnen der Informationen, die der Physiotherapie zugeordnet sind, die verbesserte aktive Bewegungsfähigkeit, die der Botulinumtoxin-Behandlung zugeordnet ist, verbessert; und
Verabreichen einer zweiten Dosis der Botulinumtoxin-Behandlung an den Patienten, wobei die Informationen, die der Physiotherapie zugeordnet sind, verwendet werden, um Art, Menge oder Häufigkeit zu bestimmen oder zu variieren oder den Muskel für die zweite Dosis der Botulinum-Neurotoxin-Behandlung auszuwählen;
wobei die Erkrankung aus Schlaganfall, Zerebralparese, Muskeldystrophie, Rückenmarksverletzung, Gehirnverletzung, einer spastischen Störung und einer neurodegenerativen Erkrankung ausgewählt ist.

2. Botulinum-Neurotoxin zur Anwendung nach Anspruch 1, wobei die Informationen, die der Physiotherapie zugeordnet sind, die Art der durchgeführten physiotherapeutischen Maßnahme; die Dauer der physiotherapeutischen Maßnahme; die Häufigkeit der physiotherapeutischen Maßnahme; die Symptome im Zusammenhang mit der beeinträchtigten aktiven Bewegungsfähigkeit, einschließlich des Ausmaßes der Schmerzen, des Ausmaßes der Steifheit, der Häufigkeit von Muskelkrämpfen, der Dauer von Muskelkrämpfen und/oder des Bewegungsumfangs; und/oder die Wirkung der physiotherapeutischen Maßnahme umfassen.

3. Botulinum-Neurotoxin zur Anwendung nach einem der vorhergehenden Ansprüche, wobei Beeinträchtigungen der aktiven Bewegungsfähigkeit des Patienten durch eine abnormale Überaktivität der Muskeln verursacht werden.

4. Botulinum-Neurotoxin zur Anwendung nach einem der vorhergehenden Ansprüche, wobei
a. das Verabreichen der Botulinum-Neurotoxin-Behandlung durch eine Injektion in die betroffenen Muskeln erfolgt;
b. der Schritt des Verabreichens der Botulinum-Neurotoxin-Behandlungen das Injizieren von Botulinum-Neurotoxin in eine neuromuskuläre Endplatte umfasst;
c. die Informationen der Physiotherapie die Art der durchgeführten körperlichen Aktivität, die Dauer der körperlichen Aktivität, die Häufigkeit der körperlichen Aktivität und/oder Symptome im Zusammenhang mit der beeinträchtigten aktiven Bewegungsfähigkeit umfassen;
d. die Informationen der Physiotherapie in einer ersten Computervorrichtung empfangen werden und in einer zweiten Computervorrichtung auf sie zugegriffen wird;
e. der Patient einen Schlaganfall erlitten hat und wobei die Verabreichung der Botulinum-Neurotoxin-Behandlung innerhalb von drei Monaten nach dem Schlaganfall erfolgt; und/oder
f. das Aufzeichnen der Informationen der Physiotherapie das Eingeben der Informationen der Physiotherapie in ein Softwareprogramm umfasst, das zum Empfangen der Informationen konfiguriert ist;
wobei optional das Softwareprogramm auf einer Computervorrichtung arbeitet;
und/oder wobei optional die Informationen der Physiotherapie in einer Datenbank gespeichert sind.

5. Botulinum-Neurotoxin zur Anwendung nach einem der vorhergehenden Ansprüche, wobei der Patient unter regelmäßiger ärztlicher Aufsicht steht.

6. Botulinum-Neurotoxin zur Anwendung nach einem der vorhergehenden Ansprüche, wobei:
das Erhalten der ersten Botulinum-Neurotoxin-Behandlung innerhalb von drei Monaten nach einem Schlaganfall erfolgt.

7. Computersystem, das programmiert ist, um Schritte eines computerimplementierten Verfahrens durchzuführen, wobei das Verfahren Folgendes umfasst:
Empfangen von Informationen, die der Physiotherapie zugeordnet sind, eines Patienten, der sich der Physiotherapie und einer Botulinum-Neurotoxin-Behandlung für eine beeinträchtigte aktive Bewegungsfähigkeit unterzieht;
Speichern der Informationen der Physiotherapie in einer Datenbank und
Übertragen der Informationen der Physiotherapie an einen Arzt, der dem Patienten die Botulinum-Neurotoxin-Behandlung verabreicht hat;
wobei der Patient eine Erkrankung hat, die der beeinträchtigten aktiven Bewegung zugeordnet ist,
wobei die Erkrankung aus Schlaganfall, Zerebralparese, Muskeldystrophie, Rückenmarksverletzung, Gehirnverletzung, einer spastischen Störung und einer neurodegenerativen Erkrankung ausgewählt ist.

8. Nichtflüchtiges computerlesbares Speichermedium, das Anweisungen speichert, die, wenn sie von einem Computersystem ausgeführt werden, bewirken, dass das Computersystem Schritte eines computerimplementierten Verfahrens durchführt, wobei das Verfahren Folgendes umfasst:
Empfangen von Informationen, die der Physiotherapie zugeordnet sind, eines Patienten, der sich der Physiotherapie und einer Botulinum-Neurotoxin-Behandlung für eine beeinträchtigte aktive Bewegungsfähigkeit unterzieht;
Speichern der Informationen der Physiotherapie in einer Datenbank und
Übertragen der Informationen der Physiotherapie an einen Arzt, der dem Patienten die Botulinum-Neurotoxin-Behandlung verabreicht hat;
wobei der Patient eine Erkrankung hat, die der beeinträchtigten aktiven Bewegung zugeordnet ist,
wobei die Erkrankung aus Schlaganfall, Zerebralparese, Muskeldystrophie, Rückenmarksverletzung, Gehirnverletzung, einer spastischen Störung und einer neurodegenerativen Erkrankung ausgewählt ist.

9. Computersystem nach Anspruch 7, wobei die Informationen der Physiotherapie von dem Computersystem einer ersten Computervorrichtung empfangen werden, sich die Datenbank auf einer zweiten Computervorrichtung befindet, die Teil des Computersystems ist, und die Informationen der Physiotherapie von dem Computersystem an eine dritte Computervorrichtung übertragen werden; wobei die erste, zweite und dritte Computervorrichtung optional jeweils separate Computervorrichtungen sind.

10. Computersystem nach Anspruch 7 oder 9, wobei das Computersystem Folgendes umfasst:
eine erste Computervorrichtung, die programmiert ist, um die Informationen der Physiotherapie des Patienten zu empfangen;
eine zweite Computervorrichtung, auf der sich die Datenbank befindet; und
eine dritte Computervorrichtung, die die übertragenen Informationen der Physiotherapie empfängt;
wobei optional die erste, zweite und dritte Computervorrichtung jeweils separate Computervorrichtungen sind;
die erste Computervorrichtung mit der zweiten Computervorrichtung in Verbindung steht und
die dritte Computervorrichtung mit der zweiten Computervorrichtung in Verbindung steht.

## Revendications

1. Neurotoxine botulique destinée à être utilisée en combinaison avec une physiothérapie dans un procédé destiné à améliorer la capacité de mouvement actif chez un sujet qui est atteint d'une affection médicale associée à un mouvement actif altéré, le procédé comprenant :
l'administration à un sujet qui en a besoin d'un premier traitement à la neurotoxine botulique, dans laquelle la neurotoxine botulique est administrée à un muscle affecté par une capacité de mouvement actif altérée ; et
(a) le fait de demander au sujet de suivre une physiothérapie qui comprend un auto-étirement quotidien du muscle affecté ; et (b) le fait de demander au sujet d'enregistrer des informations associées à la physiothérapie pour examen par un médecin ;
dans laquelle le sujet suit la physiothérapie qui comprend un auto-étirement quotidien du muscle affecté et le sujet enregistre les informations associées à la physiothérapie dans un programme logiciel tous les jours ;
dans laquelle l'enregistrement des informations associées à la physiothérapie favorise la capacité de mouvement actif améliorée associée au traitement à la toxine botulique ; et
l'administration d'une deuxième dose du traitement à la toxine botulique au sujet, dans laquelle lesdites informations associées à la physiothérapie sont utilisées pour déterminer ou faire varier le mode, la quantité, ou la fréquence ou sélectionner le muscle pour la deuxième dose du traitement à la neurotoxine botulique ;
dans laquelle ladite affection médicale est sélectionnée parmi un accident vasculaire cérébral, une paralysie cérébrale, une dystrophie musculaire, une lésion de la moelle épinière, une lésion cérébrale, un trouble spastique, et une maladie neurodégénérative.

2. Neurotoxine botulique destinée à être utilisée selon la revendication 1, dans laquelle lesdites informations associées à la physiothérapie comprennent le type d'activité de physiothérapie pratiquée ; la durée de l'activité de physiothérapie ; la fréquence de l'activité de physiothérapie ; les symptômes liés à la capacité de mouvement actif altérée incluant la quantité de douleur, la quantité de raideur, la fréquence des spasmes musculaires, la durée des spasmes musculaires, et/ou l'amplitude de mouvement ; et/ou l'effet de l'activité de physiothérapie.

3. Neurotoxine botulique destinée à être utilisée selon l'une quelconque des revendications précédentes, dans laquelle les altérations de la capacité de mouvement actif chez le sujet sont causées par une suractivité musculaire anormale.

4. Neurotoxine botulique destinée à être utilisée selon l'une quelconque des revendications précédentes, dans laquelle
a. l'administration du traitement à la neurotoxine botulique se fait par injection dans le muscle affecté ;
b. l'étape d'administration des traitements à la neurotoxine botulique comprend l'injection de neurotoxine botulique dans une jonction neuromusculaire ;
c. les informations de physiothérapie comprennent le type d'activité physique pratiquée, la durée de l'activité physique, la fréquence de l'activité physique, et/ou les symptômes liés à la capacité de mouvement actif altérée ;
d. les informations de physiothérapie sont reçues sur un premier dispositif d'ordinateur et sont accessibles sur un deuxième dispositif d'ordinateur ;
e. le sujet a souffert d'un accident vasculaire cérébral, et dans laquelle l'administration du traitement à la neurotoxine botulique a lieu dans les trois mois suivant l'accident vasculaire cérébral ; et/ou
f. l'enregistrement des informations de physiothérapie comprend la saisie des informations de physiothérapie dans un programme logiciel qui est configuré pour recevoir les informations ;
facultativement dans laquelle le programme logiciel fonctionne sur un dispositif d'ordinateur ;
et/ou facultativement dans laquelle les informations de physiothérapie sont stockées dans une base de données.

5. Neurotoxine botulique destinée à être utilisée selon l'une quelconque des revendications précédentes, dans laquelle le sujet est guidé régulièrement par un médecin.

6. Neurotoxine botulique destinée à être utilisée selon l'une quelconque des revendications précédentes, dans laquelle :
la réception du premier traitement à la neurotoxine botulique a lieu dans les trois mois suivant un accident vasculaire cérébral.

7. Système d'ordinateur programmé pour effectuer des étapes d'un procédé mis en œuvre par ordinateur, le procédé comprenant :
la réception d'informations associées à une physiothérapie, d'un sujet suivant ladite physiothérapie et un traitement à la neurotoxine botulique pour une capacité de mouvement actif altérée ;
le stockage des informations de physiothérapie dans une base de données ; et
la transmission des informations de physiothérapie à un médecin qui a administré le traitement à la neurotoxine botulique au sujet ;
dans lequel le sujet est atteint d'une affection médicale associée audit mouvement actif altéré,
dans lequel ladite affection médicale est sélectionnée parmi un accident vasculaire cérébral, une paralysie cérébrale, une dystrophie musculaire, une lésion de la moelle épinière, une lésion cérébrale, un trouble spastique, et une maladie neurodégénérative.

8. Support de stockage lisible par ordinateur non transitoire stockant des instructions qui, lorsqu'elles sont exécutées par un système d'ordinateur, amènent le système d'ordinateur à effectuer des étapes d'un procédé mis en œuvre par ordinateur, le procédé comprenant :
la réception d'informations associées à une physiothérapie, d'un sujet suivant ladite physiothérapie et un traitement à la neurotoxine botulique pour une capacité de mouvement actif altérée ;
le stockage des informations de physiothérapie dans une base de données ; et
la transmission des informations de physiothérapie à un médecin qui a administré le traitement à la neurotoxine botulique au sujet ;
dans lequel le sujet est atteint d'une affection médicale associée audit mouvement actif altéré,
dans lequel ladite affection médicale est sélectionnée parmi un accident vasculaire cérébral, une paralysie cérébrale, une dystrophie musculaire, une lésion de la moelle épinière, une lésion cérébrale, un trouble spastique, et une maladie neurodégénérative.

9. Système d'ordinateur selon la revendication 7, dans lequel les informations de physiothérapie sont reçues par le système d'ordinateur à partir d'un premier dispositif d'ordinateur, la base de données réside sur un deuxième dispositif d'ordinateur qui fait partie du système d'ordinateur, et les informations de physiothérapie sont transmises par le système d'ordinateur à un troisième dispositif d'ordinateur ; facultativement dans lequel les premier, deuxième, et troisième dispositifs d'ordinateur sont chacun des dispositifs d'ordinateur distincts.

10. Système d'ordinateur selon la revendication 7 ou 9, dans lequel le système d'ordinateur comprend :
un premier dispositif d'ordinateur qui est programmé pour recevoir les informations de physiothérapie du sujet ;
un deuxième dispositif d'ordinateur sur lequel réside la base de données ; et
un troisième dispositif d'ordinateur qui reçoit les informations de physiothérapie transmises ; facultativement
dans lequel les premier, deuxième, et troisième dispositifs d'ordinateur sont chacun des dispositifs d'ordinateur distincts ;
le premier dispositif d'ordinateur est en communication avec le deuxième dispositif d'ordinateur ; et
le troisième dispositif d'ordinateur est en communication avec le deuxième dispositif d'ordinateur.
